# EUROPEAN PATENT SPECIFICATION

(11) **EP 2 313 137 B1**
(45) Date of publication and mention of the grant of the patent: **11.10.2017**
(21) Application number: 09805221.0
(22) Date of filing: 04.08.2009
(51) Int. Cl.: A61M 16/06, A61M 16/00, A61M 16/16

(54) **RESPIRATORY MASK SEALING INTERFACE**
VERSCHLUSSSCHNITTSTELLE FÜR EINE ATEMMASKE
INTERFACE DE FIXATION D'UN MASQUE RESPIRATOIRE

(30) Priority: 04.08.2008 US 86051 P
(43) Date of publication of application: 27.04.2011
(60) Divisional application: 17184566.2
(73) Proprietor: Fisher & Paykel Healthcare Limited, 2012 Manukau (NZ)
(72) Inventor: MCAULEY, Alastair, Edwin, Auckland 1050 (NZ); MCLAREN, Mark, Manukau 2014 (NZ); HUANG, Wen, Dong, Manukau 2010 (NZ)
(74) Representative: Hoarton, Lloyd Douglas Charles
(86) International application number: PCT/NZ2009/000157
(87) International publication number: WO 2010/016774

(56) References cited:
- WO-A1-2005/053781
- WO-A1-2005/053781
- WO-A1-2005/118040
- WO-A1-2005/118040
- WO-A1-2005/123166
- WO-A1-2006/074513
- US-B2- 7 308 895

## Description

### FIELD OF THE INVENTION

The present invention relates to a patient sealing interface for use as part of an apparatus for supplying a flow of respiratory gases to a user.

### BACKGROUND TO THE INVENTION

It is known to provide a flow of respiratory gases to a user via an interface such as a face mask to relieve a number of ailments - for example sleep apnea or snoring. One problem with supplying a flow of gases to a user via an interface such as a face mask is that it can be difficult to form a good seal between the mask and the face. The mask is often held in place against the user's face by headgear worn on the user's head. In use, the head gear may be over tightened so that the mask is pressed uncomfortably onto the user's face. Alternatively the headgear may be under tightened or applied to the user's head too loosely, preventing the formation of an effective seal between the mask and user's face.

Prior art face masks have attempted to improve the seal between the user's face and the mask and make the sealing interface with the user more comfortable. US 7,308,895 describes a mask assembly having a seal outer sheath and inner cushion. The inner cushion has a raised nasal bridge portion which results in a more flexible seal contact on the bridge of the user's nose. The raised nasal bridge portion is formed by a cut out portion of the inner cushion, the cut-out being on a mask body side of the cushion.

US 6,112,746 describes a nasal mask cushion for sealing a nasal mask to a user's face. The cushion has a first membrane and a second membrane. The second membrane contacts a user's face when in use. The second membrane is thinner than the first membrane and is spaced apart from the first membrane when the mask is not in use. The second membrane is spaced from the first membrane by a greater distance in the nasal bridge region than in the cheek region.

WO2005/053781 describes a nasal mask with an inner cushion and ah outer sheath. In one embodiments the inner cushion has an outer film. In another embodiment the inner cushion has a honeycomb like structure.

WO 2005/118040 describes an integrally formed cushion for a patient interface comprising an inner membrane and an outer membrane. In one embodiment the inner membrane is segmented along its inner edge to provide fingers interconnected by webbing.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

### SUMMARY OF THE INVENTION

It is an object of the present invention to provide an improved patient sealing interface, or to at least provide the industry or the public with a useful choice.

The present invention provides a sealing interface (100) for use as part of an apparatus for supplying a flow of respiratory gases to a user, as claimed.

Preferably the toothed profile extends substantially around the full perimeter of the inner cushion first side.

Preferably the sheath first side and the cushion first side have a nasal bridge region, either one of an upper lip region or a chin region and a left cheek region and a right cheek region extending between the nasal bridge region and the upper lip or chin region, and a said toothed profile is in the upper lip region or chin region.

Preferably a said toothed profile is in a lower portion of each of the left and right cheek regions.

Preferably a said toothed profile is in nasal bridge region.

Preferably the at least one tooth has at least two converging sides, each said converging side having an angle of convergence, the angle of convergence being at least 30 degrees.

Preferably the tooth has a depth, the depth being approximately 3mm to 10mm.

Preferably the tooth has a base, the base being approximately 2mm to 20mm wide.

Preferably the at least one tooth is aligned inwards towards a centre of the inner cushion, a side of the at least one tooth being coterminous with the face side of the inner cushion and an opposite side of the at least one tooth being conterminous with an inside surface of a perimeter wall of the inner cushion, an apex of said at least one tooth being formed where the inside surface of the perimeter wall meets the face side of the inner cushion.

Preferably the toothed profile comprises a plurality of teeth, in use each said tooth having an apex in supporting contact with the sheath first side.

Preferably each said tooth has an apex, a distance between the apex of adjacent teeth being less than approximately 20mm.

Preferably each said tooth has a depth, and a ratio of the tooth depth over the tooth pitch is at least approximately 0.3, wherein the pitch is the distance between the apexes of adjacent teeth.

Preferably the tooth depth decreases as the toothed profile extends from a central position of the toothed profile to a side of the toothed profile extending along the perimeter of the sealing interface.

Preferably the inner cushion comprises a plurality of spaced apart cushions, as said cushion being a tooth of the toothed profile.

Preferably the spaced apart cushions are joined by joining elements.

Preferably the inner cushion has at least two holes through a perimeter wall of the inner cushion, the at least two holes breaking the face side of the inner cushion to create the at least one tooth between said holes.

Also described is a sealing interface for use as part of an apparatus for supplying a flow of respiratory gases to a user comprising:
a thin resilient rubber outer sheath and a foam or gel inner cushion, in use the outer sheath covering the inner cushion , the outer sheath having a sheath first side, the sheath first side substantially sealing against a user's face, a face side of the inner cushion resiliently supporting at least a portion of the sheath first side,
the inner cushion having a perimeter wall, and at least two holes through the perimeter wall adjacent the face side of the inner cushion.

Further described is a sealing interface for use as part of an apparatus for supplying a flow of respiratory gases to a user comprising:
a thin resilient rubber outer sheath and a foam or gel inner cushion, in use the outer sheath covering the inner cushion , the outer sheath having a sheath first side, the sheath first side substantially sealing against a user's face, a face side of the inner cushion resiliently supporting at least a portion of the sheath first side, and
at least two cavities in the face side of the inner cushion.
The present invention also provides a mask assembly for use as part of an apparatus for supplying a flow of respiratory gases to a user comprising:
a mask body having an inlet through which said flow of respiratory gases are provided to the interior of said mask body, the inlet adapted to in use be connected to a gases conduit, and
a sealing interface coupled to the mask body, according to claim 13.

The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner.

This invention may also be said broadly to consist in the parts, elements and features referred to or indicated in the specification of the application, individually or collectively, and any or all combinations of any two or more said parts, elements or features, and where specific integers are mentioned herein which have known equivalents in the art to which this invention relates, such known equivalents are deemed to be incorporated herein as if individually set forth.

### BRIEF DESCRIPTION OF THE DRAWINGS

Preferred embodiments of the invention will be described by way of example only and with reference to the drawings, in which:
**Figure 1** is a block diagram of a system for providing a heated humidified gases stream to a user such as a continuous positive airway pressure system as might be used in conjunction with the patient sealing interface of the present invention.
**Figure 2** is a diagram of a nasal mask that may incorporate the sealing interface of the present invention.
**Figure 3** is a cross sectional view of a face mask that may incorporate the sealing interface of the present invention.
**Figure 4a** is a perspective view of a prior art sealing interface inner cushion.
**Figure 4b** is a side view of the prior art sealing interface inner cushion of Figure 4a viewed in the direction of arrow A.
**Figure 4c** is an end view of the prior art sealing interface inner cushion of Figure 4a viewed in the direction of arrow B.
**Figure 5a** is a sealing interface inner cushion according to a first embodiment of the present invention.
**Figure 5b** is a sealing interface inner cushion according to a second embodiment of the present invention.
**Figures 6a - 6e** are bottom views of a face mask incorporating various embodiments of the sealing interface of the present invention.
**Figure 6f** is a bottom view of a face mask with a prior art sealing interface arrangement with a gap between an inner cushion and an outer sheath.
**Figures 7a and 7b** show bottom views of a face mask incorporating one embodiment of the sealing interface of the present invention, with the chin and jaw line of two users indicated.
**Figure 8a - 8j** show perspective views of various embodiments of a tooth profile for a toothed portion of the sealing interface cushion of the present invention.
**Figure 8k** is a side view of the tooth shape of Figure 8a viewed in the direction of arrow D.
**Figure 9** is a perspective view of a preferred embodiment of the sealing interface cushion of the present invention.
**Figure 10a** is a perspective view of an alternative embodiment of the sealing interface cushion of the present invention.
**Figure 10b** is a bottom view of the sealing interface cushion of Figure 9a viewed in the direction of arrow C.
**Figure 11** is a chart showing data relating to leak rates comparing a prior art mask sealing interface and a sealing interface incorporating a sealing interface cushion of the present invention.
**Figures 12a** and **12b** are charts showing a difference in compression force required to compress the sealing interface cushion of the present invention.
**Figures 13a** and **13b** are perspective views of alternative embodiments of the sealing interface cushion of the present invention incorporating a hole profile with holes through a wall of the inner cushion.
**Figures 14a****,** **14c and 14d** are perspective views of alternative embodiments of the sealing interface cushion of the present invention incorporating holes or cavities in a side of the sealing interface cushion that faces a user's face in use.
**Figure 14b** is a cross sectional view of the sealing interface cushion of Figure 14a on arrows A.
**Figures 15a, 15b****,** **15d and 15e** are perspective views of alternative embodiments of the present invention where the sealing interface cushion comprises a plurality of spaced apart cushions.
**Figure 15c** is a perspective view of an alternative embodiment of the present invention where the sealing interface cushion comprises spaced apart cushions joined together by joining elements.

### DETAILED DESCRIPTION

The sealing interface of the present invention provides improvements in the delivery of CPAP therapy. In particular a sealing interface is described which reduces the pressure of the mask on the users face and reduces leakage when compared with the prior art. It will be appreciated that the sealing interface as described in the preferred embodiment of the present invention can be used in respiratory care generally or with a ventilator but will now be described below with reference to use in a humidified CPAP system. It will also be appreciated that the present invention can be applied to any form of patient interface including, but not limited to, full face masks sealing around the user's nose and mouth, nasal masks sealing around the user's nose, and oral masks and mouthpieces in sealing engagement with the user's mouth.

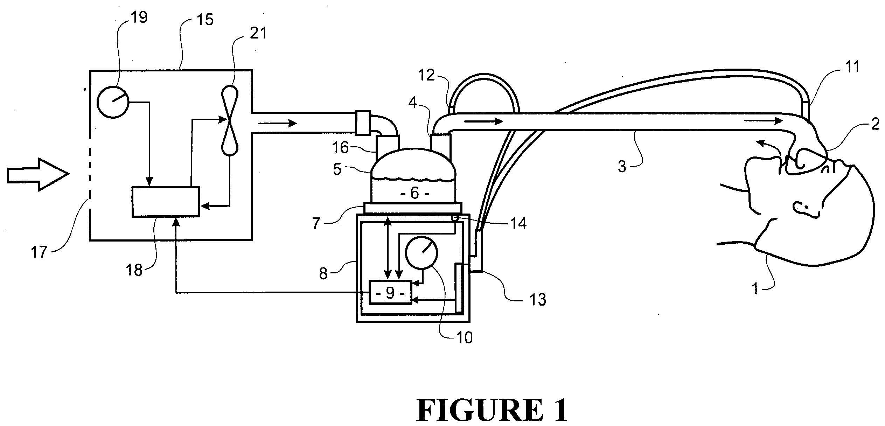

With reference to Figure 1 a humidified Continuous Positive Airway Pressure (CPAP) system is shown in which a patient 1 is receiving humidified and pressurised gases through a patient interface 2 connected to a humidified gases transportation pathway or inspiratory conduit 3. It should be understood that delivery systems could also be VPAP (Variable Positive Airway Pressure) and BiPAP (Bi-level Positive Airway Pressure) or numerous other forms of respiratory therapy. Inspiratory conduit 3 is connected to the outlet 4 of a humidification chamber 5 which contains a volume of water 6. Inspiratory conduit 3 may contain heating means or heater wires (not shown) which heat the walls of the conduit to reduce condensation of humidified gases within the conduit. Humidification chamber 5 is preferably formed from a plastics material and may have a highly heat conductive base (for example an aluminium base) which is in direct contact with a heater plate 7 of humidifier 8. Humidifier 8 is provided with control means or electronic controller 9 which may comprise a microprocessor based controller executing computer software commands stored in associated memory.

Controller 9 receives input from sources such as user input means or dial 10 through which a user of the device may, for example, set a predetermined required value (preset value) of humidity or temperature of the gases supplied to patient 1. The controller may also receive input from other sources, for example temperature and/or flow velocity sensors 11 and 12 through connector 13 and heater plate temperature sensor 14. In response to the user set humidity or temperature value input via dial 10 and the other inputs, controller 9 determines when (or to what level) to energise heater plate 7 to heat the water 6 within humidification chamber 5. As the volume of water 6 within humidification chamber 5 is heated, water vapour begins to fill the volume of the chamber above the water's surface and is passed out of the humidification chamber 5 outlet 4 with the flow of gases (for example air) provided from a gases supply means or blower 15 which enters the chamber through inlet 16. Exhaled gases from the patient's mouth are passed directly to ambient surroundings in Figure 1.

Blower 15 is provided with variable pressure regulating means or variable speed fan 21 which draws air or other gases through blower inlet 17. The speed of variable speed fan 21 is controlled by electronic controller 18 (or alternatively the function of controller 18 could carried out by controller 9) in response to inputs from controller 9 and a user set predetermined required value (preset value) of pressure or fan speed via dial 19.

### Patient Interface

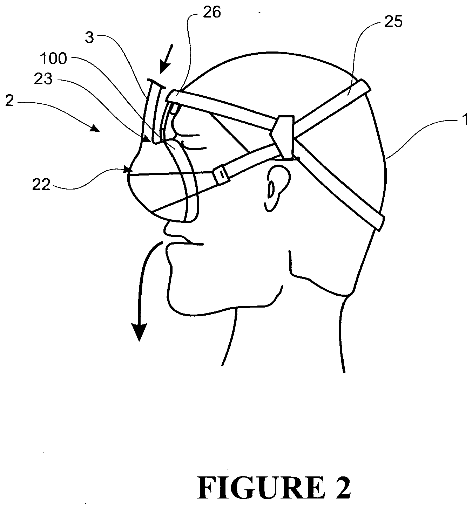

A typical patient interface in the form of a nasal mask is shown in Figure 2. The mask includes a hollow body 22 with an inlet 23 connected to the inspiratory conduit 3. The mask 2 is positioned around the nose of the user 1 with the headgear 25 secured around the back of the head of the patient 1. The restraining force from the headgear 25 on the hollow body 22 and the forehead rest 26 ensures enough compressive force on the mask seal 100, to provide an effective seal against the patient's face.

The hollow body 22 is constructed of a relatively inflexible material for example, polycarbonate plastic. Such a material would provide the requisite rigidity as well as being transparent and a relatively good insulator. The expiratory gases can be expelled through a valve (not shown) in the mask, a further expiratory conduit (not shown), vent paths through the mask, or any other such method as is known in the art.

### Mask Seal

The mask seal 100 is provided around the periphery of the mask body 22 to provide an effective seal onto the face of the user to prevent leakage. The mask seal 100 is shaped to approximately follow the contours of a patient's face. The seal is contoured to approximately match the facial contours of a user around the user's nose, from the bridge of the nose, continuing down the cheek regions adjacent each side of the user's nose and across the user's philtrum area. Similarly, if the seal was applied to a full face mask covering a user's nose and mouth, the face seal would be shaped to approximate the facial contours of the user's chin and wider cheek regions. The mask seal 100 will deform when pressure is applied by the headgear 25 to adapt to the individual contours of most users.

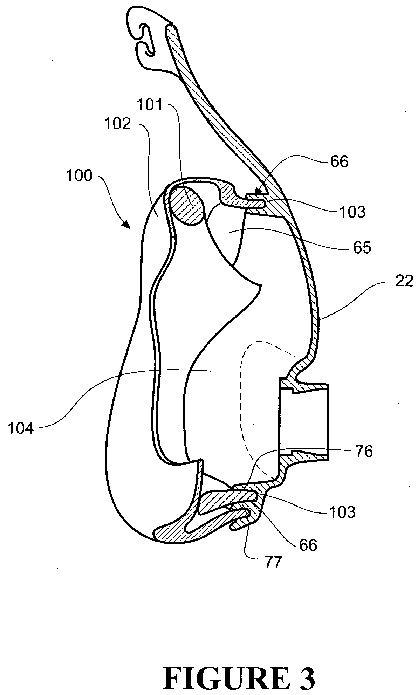

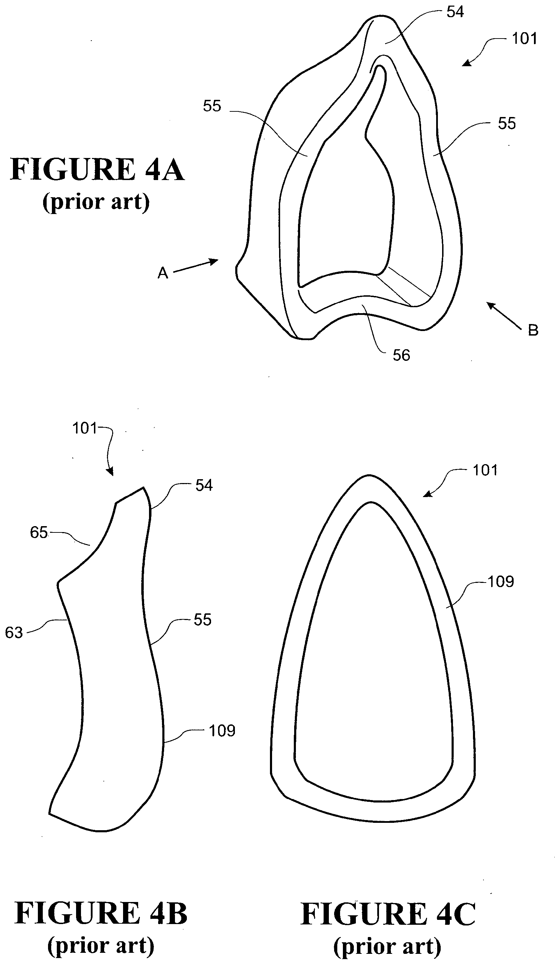

A prior art full face mask assembly and mask seal arrangement for sealing around a user's nose and mouth is shown in Figure 3. The mask seal 100 is composed of an inner foam cushion 101 covered by an outer sealing sheath 102. The inner cushion 101 is constructed of a resilient material, for example polyurethane foam, to distribute the pressure along the seal around the user's face. In other forms the cushion 101 may be formed of other appropriate material, such as a gel material. One side of the inner cushion is shaped to approximately match the shape of the user's face. As shown in Figure 4, there is an indented section 54 intended to fit over the bridge of the patient's nose, a cheek contour 55 on each side to follow the cartilage extending from the middle of the user's nose, and an indented section 56 to seal around the chin area of the user. An opposite side of the cushion 63 is shaped to match and interface to the mask body.

The inner cushion may include a raised bridge 65 in the nasal bridge region. The raised bridge 65 can also be described as a valley formed in the cushion on the mask body side 63 of the cushion. As the raised bridge 65 is unsupported by the mask body 22, it is much more flexible and results in less pressure on the nasal bridge of the patient.

In other forms, the cushion may have other bridge portions, so that in these bridging areas the cushion is more flexible.

The inner cushion 101 is located around an inner periphery 103 of the open face 104 of the hollow body 22, contacting the mask body except for in the raised bridge portion 65. As best shown in Figure 3, the cushion is located in a cavity 66 extending around the inner periphery 103 of the body 22, terminating at each side of the nose bridge region 67 of the mask, where the raised bridge portion 65 of the cushion does not contact the mask body 22. The cavity 66 is generally formed by two spaced apart walls 76 and 77 extending around the inner periphery of the mask.

Similarly the outer sheath 102 is attached to an outer periphery of the mask body 22, either directly to the body 22 in a push fit arrangement as shown in Figure 3, or indirectly via a mask seal clip (not shown). Preferably a side of the seal 102 is attached to a seal clip (not shown). The seal clip interfaces to the mask body 22. The clip provides a releasable rigid or semi rigid interface, to allow the seal to be easily attached and detached from the mask body many times. The outer sheath 102 surrounds and loosely covers over the top of the inner cushion 101.

One side of the outer sheath is also shaped to match the facial contours of a user's face, and closely matches the shape of the side of the cushion adjacent a user's face in use.

Preferably the cushion 101 is a separate item; the outer sheath 102 fitting in place over the cushion 101. In the preferred embodiment, the outer sheath holds the cushion in place within the mask assembly 2. Alternatively, the cushion may be permanently or releasably attached to the outer sheath so that the outer sheath and cushion may be provided as a single assembly. Alternatively, the cushion may be permanently or releasably attached to the mask body 22. In a further alternative, the outer sheath and inner cushion may be integrally formed.

### Toothed Profile

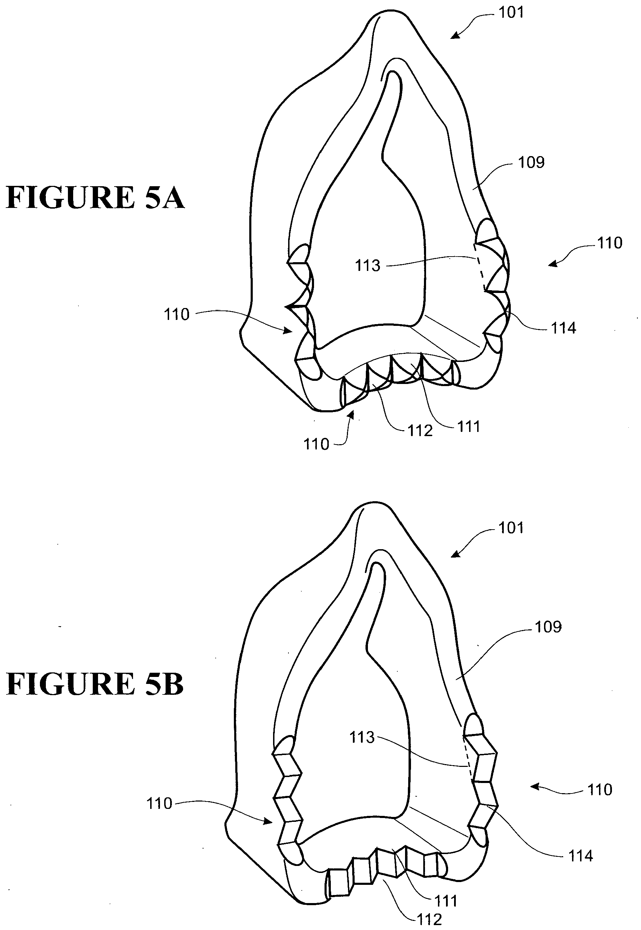

As shown in Figures 5 to 7, the present invention is exemplified in a mask cushion for use in a mask assembly such as the prior art mask assembly described above. Like the inner cushion of the prior art sealing interface, the inner cushion 101 according to the present invention is constructed of a resilient material, preferably polyurethane foam, to distribute the pressure along the seal around the user's face. In other forms the cushion 101 may be formed of other appropriate material, such as a gel material. The cushion may be formed from any suitable resilient material having similar stiffness to a two part polyurethane foam with a density of approximately 0.20 - 0.25g/cm³.

The outer sheath is a thin resilient rubber material for forming a seal against the user's face in use. Preferably the outer sheath is formed from silicone.

The mask cushion according to one embodiment of the present invention has a toothed profile 110 on the side of the cushion 101 that bears against a user's face when in use, the outer sheath 102 being located between the user's face and the toothed profile when in use. One significant advantage of the present invention is that the valleys 112 of the toothed profile 110 are on the user's face side 109 of the cushion. The inventors have found that having the toothed profile 110 on the face side of the cushion results in an improved seal for a given sealing force: To create an effective seal on the users face, the head gear 25 is tightened sufficiently to compress the cushion 101 against the face. The toothed profile helps achieve an effective seal using a lower sealing force than a similar inner cushion without a toothed profile. This results in a more comfortable patient interface. A user can use the mask 2 with a lower sealing force, with the headgear and mask assembly applied to the head and face with less tension in the head gear straps and lower compressive force of the seal 100 on the face. A lower force results in less irritation over an extended use period. Patients are more likely to use the mask and adhere to treatment requirements.

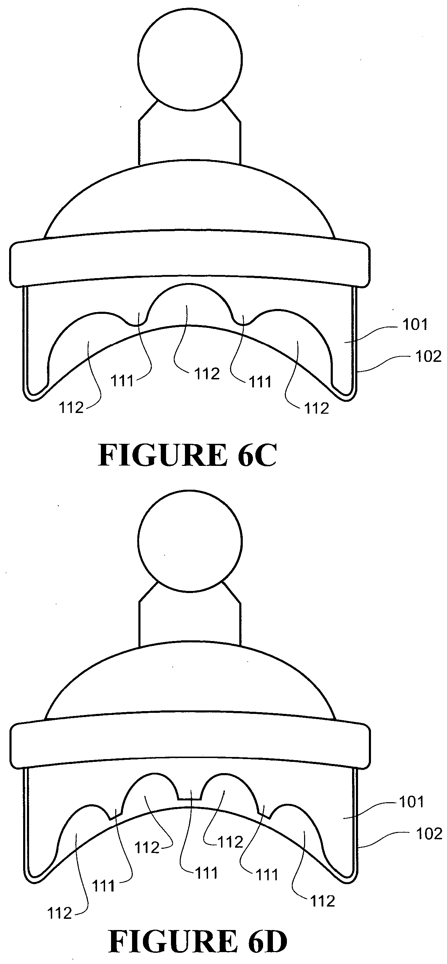

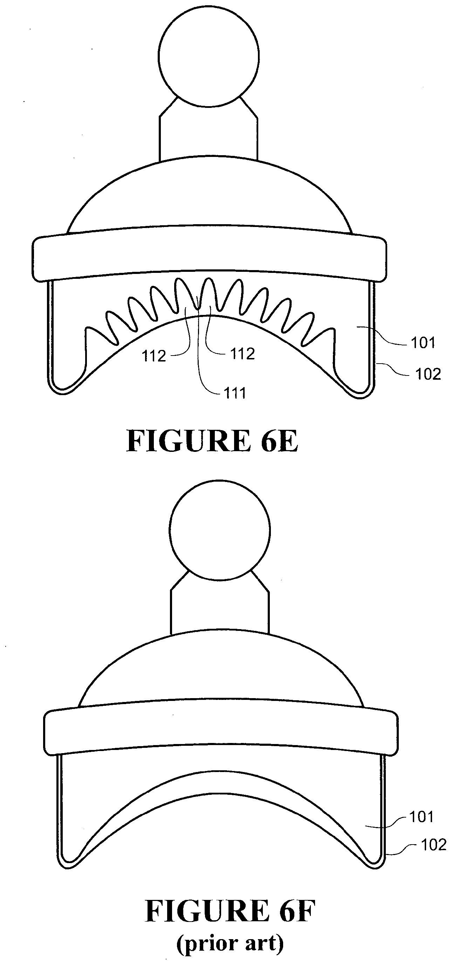

Some prior art masks have a gap between the outer sheath and the inner cushion as shown in Figure 6f. The mask of Figure 6f is shown when not in use. In use, the gap between the outer sheath and the inner cushion may or may not close depending on the sealing force applied to the sealing interface. However, the inventors have found that an improvement is achieved by providing discrete points or areas 114 of support for the outer sheath when in use. The contact support points or areas are spaced apart along the perimeter of the mask sealing interface.

The toothed profile consists of at least one tooth 111 having an apex 114 on the perimeter of the cushion. The apex is positioned between two valleys 112 in the perimeter of the cushion. The at least one tooth 111 is formed in the face side 109 of the cushion.

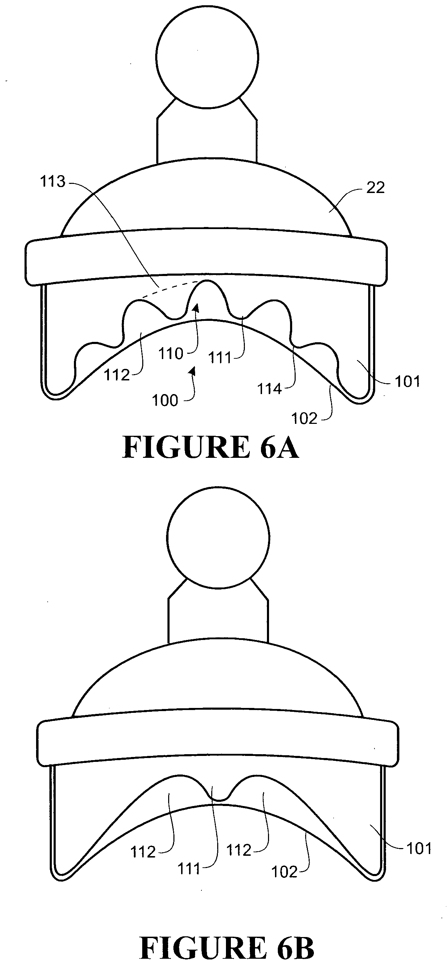

The at least one tooth has a base 113, indicated by the dashed line in Figure 6a, and an apex 114. The toothed profile achieves an improved seal by allowing for an increased amount of compression of the cushion for a given force, due to the reduced bearing surface area of the face side of the cushion compared to a cushion having no valleys 112. Furthermore, the apex 114 of the at least one tooth may deflect relatively easily in any direction laterally across the face, that is, vertically on the face, horizontally on the face, or any other direction across the face. In the prior art cushion of Figure 3, a particular point on the face side of the cushion cannot deflect sideways as easily due to support provided in the cushion material adjacent that point. Having contact points or areas 114 in contact with the outer sheath provides improved flexibility of the cushion and an improved level of fit for a given sealing force.

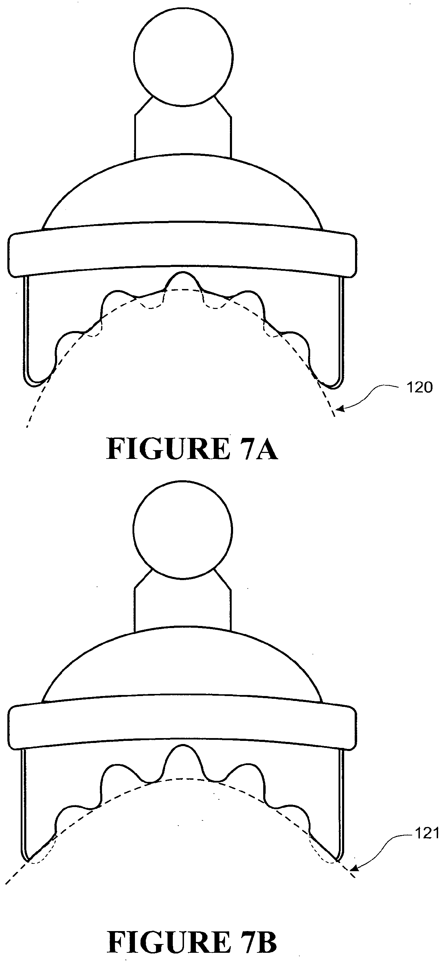

Due to the different facial contours of different users, to achieve an effective seal on any given user's face the seal should conform to a particular user's face easily. For example, when considering the shape of the chin region, one user's chin may be relatively broad and another user's chin may be relatively narrow. As shown in Figure 7a, a user's chin and jaw line is indicated by the dashed line 120. In order to conform to line 120, each tooth must be significantly compressed. Due to the reduced bearing area of the face side of the cushion comprising a toothed profile, the force required to press the seal 100 against the users face is lower than for a cushion without a toothed profile. Figure 7b shows the chin and jaw line of a user with a broader chin and jaw line 121 than the jaw line indicated by line 120. In order to conform to a broader chin, the toothed profile does not require as much compression. However, the apex 114 of each tooth 111 of the toothed profile 110 provides contact support against the outer sheath at positions around the periphery of the mask seal 100. The toothed profile provides more contact points than the prior art seal arrangement of Figure 6f, and these contact points deflect and compress more easily than a prior art inner cushion without a toothed profile. Teeth that require a relatively high level of compression to conform to a user's face, as shown in Figure 7a, will compress more easily compared to a cushion without a toothed profile. Teeth that require a relatively low level of compression or substantially no compression, as shown in Figure 7b may still maintain contact with and provide support to the outer sheath. The toothed profile therefore provides an improved fit against a range of user's with different facial contours.

The toothed profile achieves points of contact between the inner cushion and outer sheath along the sealing interface perimeter. As the sealing interface is compressed against the face, the outer sheath is stretched over the contact points and bridges the gaps between contact points. The seal, at positions where the outer sheath bridges valleys between teeth contact points, is relatively easy to deflect or compress a given amount of compression. The seal, where teeth contact the outer sheath, requires a higher level of force to compress for the same amount of compression.

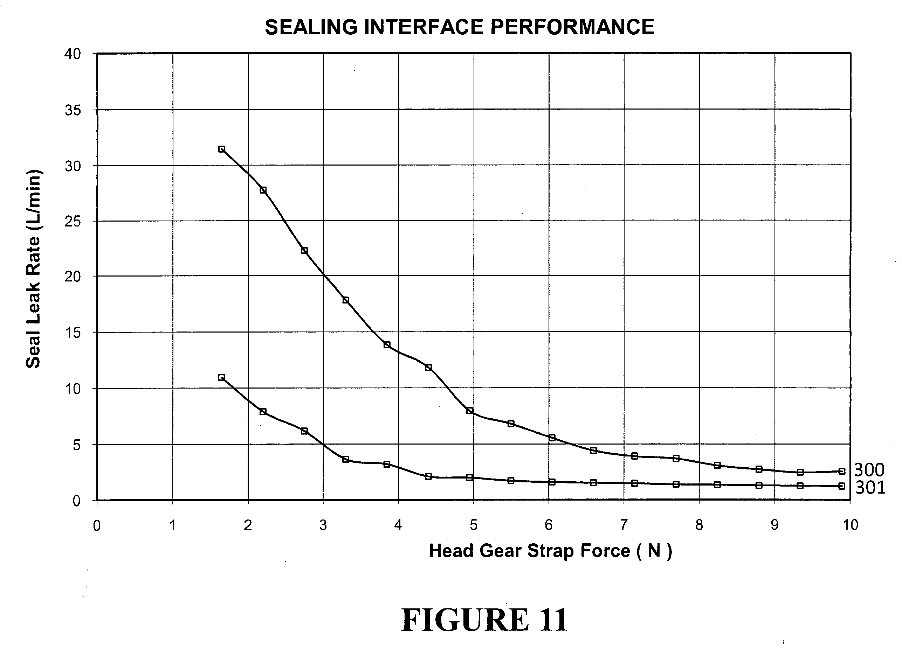

The improvement in sealing efficiency of a seal interface incorporating a toothed inner cushion is indicated in Figure 11. Figure 11 is a chart displaying the sealing efficiency of a sealing interface incorporating an inner cushion with a toothed profile, compared to a sealing interface incorporating the same inner cushion but without a toothed profile. From Figure 11 it can be seen that a lower headgear strap force (lower tension in strap), that is a lower mask seal force against a user's face, is required to achieve a given leak rate. The trend line in Figure 11 labelled 300 reflects data collected for a sealing interface comprising an inner cushion without a toothed profile. The trend line in Figure 11 labelled 301 reflects data collected for a sealing interface comprising an inner cushion with a toothed profile in the face side of the inner cushion. For example, for a leak rate of 10 litres per minute, the toothed cushion sealing interface requires a strap tension of less than 2 Newtons. Conversely, the same mask assembly incorporating the same inner cushion but without a toothed profile requires more than 4 Newtons of strap force to reduce the leak rate to 10 litres per minute. The data presented in Figure 11 is averaged data collected from a range of different facial shapes.

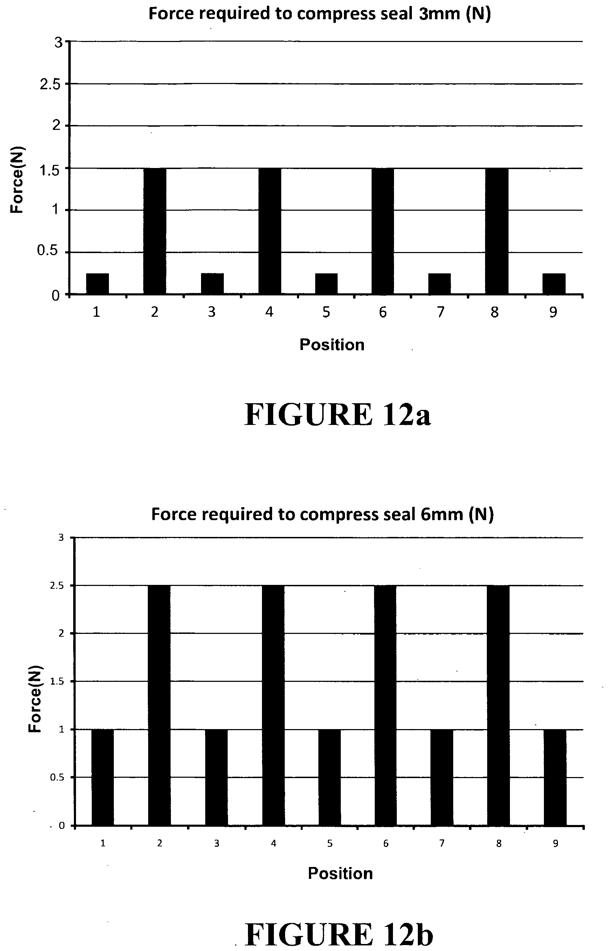

The effect of the toothed profile can be seen in Figures 12a and 12b. A Newton meter fitted with a cone tip was used to measure the force required to compress the sheath and cushion of seal 100 by a set distance. The cone tip used with the Newton meter had a base diameter of approximately 10mm and a height of approximately 5mm, the angle between the side of the cone and a plane perpendicular to the cone axis being about 45 degrees. The bars of each chart labelled with even numbers relate to positions along the mask seal at the apexes 114 of the toothed profile of the mask cushion 101. The bars labelled with odd numbers relate to positions along the mask seal 100 in between the apexes of the teeth of the toothed profile.

Figure 12a shows a bar chart of the force required to compress different positions of the mask seal 100 by 3mm. To compress the seal by 3mm at the apex of a tooth requires a force of 1.5N. To compress the seal by 3mm at positions where the sheath is initially unsupported by the cushion 101, where the sheath is located over valleys 112 of the mask cushion 101, requires a force of around 0.25N.

Figure 12b shows the same bar chart as Figure 12a but for a compression of the mask seal 100 by 6mm.

The force required to compress a point on a seal 100 comprising a cushion without teeth is similar to the force required to compress a seal at the apex 114 of a tooth for a seal with a cushion 101 having a toothed profile 110. For example, to compress a point on a seal, the seal having a sheath and cushion without teeth, by 6mm will require approximately 2.5N of force.

The charts of Figures 12a and 12b show how the overall force required to compress the seal 100 of the mask is reduced with the introduction of the toothed profile, while the toothed profile maintains support for the cushion at different points along the perimeter of the seal. Also, charts 12a and 12b show how the difference between forces at different points change with increased compression. As the amount of compression of seal 100 is increased, the ratio of the force to compress the seal at a tooth over the force to compress the seal at a cushion gap 112 reduces. For example, for 3mm compression of Figure 12a, the ratio is 6, that is, the force required to compress the seal at a tooth is six times the force required to compress the seal at a tooth gap, with the outer sheath in contact with the cushion at the apexes of the toothed profile. And for a compression of 6mm, the force required to compress the seal at a tooth is 2.5 times the force required to compress the seal at a tooth gap, with the outer sheath in contact with the cushion at the apexes of the toothed profile. As the amount of seal compression increases further, the ratio tends towards 1.

Figures 6a to 6e are bottom views of face masks incorporating various embodiments of the sealing interface 100 of the present invention with the sealing interface in the uncompressed state. The sealing interface 100 is in the uncompressed state when not being used. Figures 6a to 6e show the sheath 102 and cushion 101 spaced apart when in the uncompressed state. However, the apex 114 of any one or more teeth 111 may contact the outer sheath when the interface seal is in the uncompressed state, with valley areas 112 not contacting the outer sheath.

The toothed profile 110 may comprise a single tooth, as shown in Figure 6b. Alternative embodiments may include two, three, or many teeth, as shown in Figures 6c to 6e. The preferred embodiment has a toothed profile in the chin region consisting of four teeth, as shown in Figure 6a. Each tooth is formed on the side 109 of the cushion that bears against a user's face when in use.

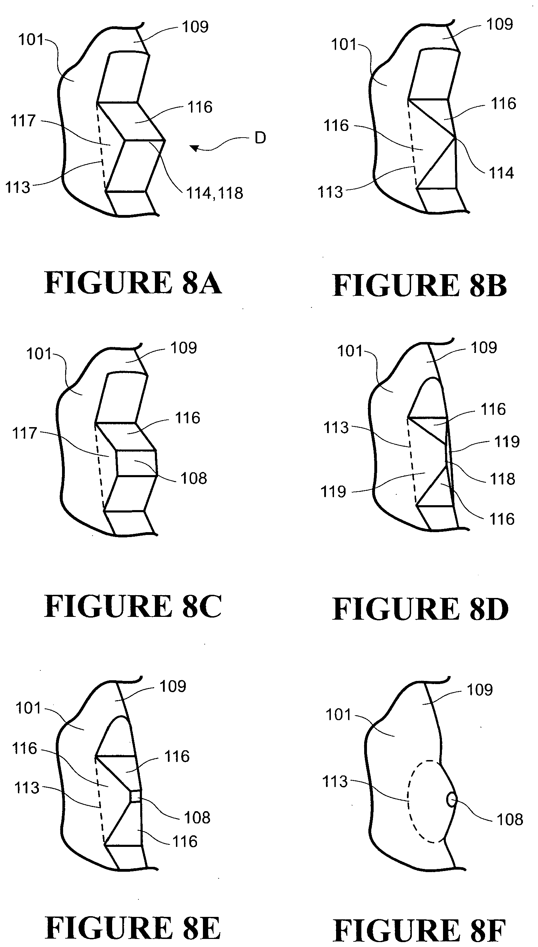

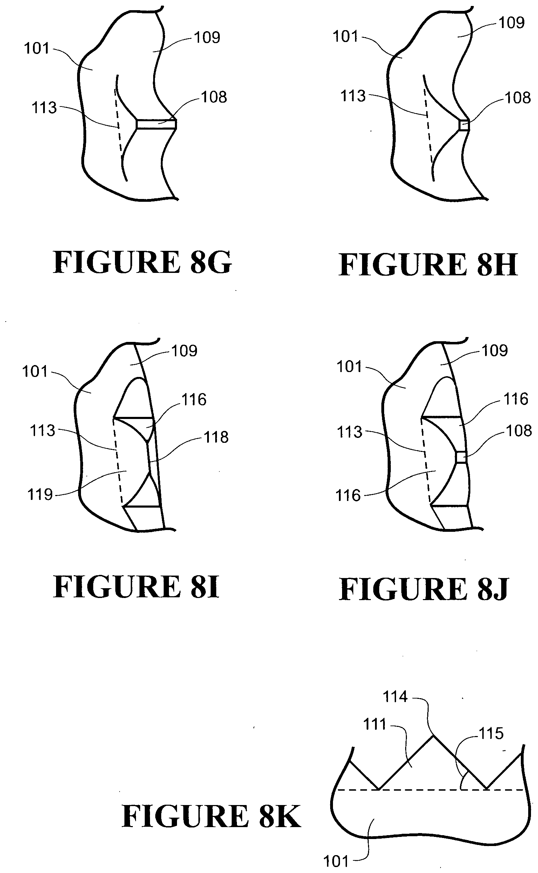

Various tooth profiles are shown in Figures 8a to 8j. The at least one tooth preferably has four sides 116 as shown in Figures 8b, 8d, 8e, 8h, 8i and 8j, the four sides converging from the base 113 to the apex 114. The apex may be formed by the four sides 116 converging to a point, as shown in Figure 8b. Alternatively, the tooth may have two converging sides and two substantially parallel sides 117 as shown in Figures 8a, 8c and 8g, such that the apex is formed as a ridge 108, 118. The inner cushion of Figure 5b comprises a toothed profile 110 with teeth 111 having a profile similar to the profile of Figure 8a. Alternatively the tooth may have four converging sides, with two opposite sides 119 converging at a lower angle of convergence compared to the other two opposite sides 116, such that the apex is formed as a ridge 118, as shown in Figures 8d and 8i. Alternatively, the base 113 of the tooth may be substantially rectangular, such that a ridge apex 118 is formed with four sides converging at the same angle.

Preferably the apex 114, 118 is truncated to form an apex having an area 108 substantially reduced compared to the area of the base 113, as shown in Figures 8c, 8e, 8g, 8h and 8j. The inner cushion of Figure 5a comprises a toothed profile 110 with teeth 111 in a lower portion of the cheek regions 55 of the inner cushion 101 having a profile similar to the profile of Figure 8j. Alternatively the tooth may be substantially frustoconical in shape, as indicated in Figure 8f. Apex area 108 of the tooth of Figure 8f may be rounded, similar to the tooth profile of Figure 8j.

The tooth profiles of Figures 8a to 8j may be symmetrical about a first plane and a second plane, the first plane being in line with the ridge 118 of Figure 8a and the second plane being in line with the ridge 118 of Figure 8d. Alternatively, the tooth 111 may have an asymmetrical shape, being symmetrical about one plane only, with the apex 114, 119 offset to one side of the tooth 111. The inner cushion of Figure 5a comprises a toothed profile 110 with teeth 111 in a chin region 56 of the inner cushion 101, the teeth having a shape with the apex 114 being offset towards an outside surface of the inner cushion 101 (outside surface at the bottom of the cushion obstructed from view in Figure 5a). Alternatively, the toothed profile 110 may comprise teeth having a shape with the apex 114,119 offset to two sides of the tooth 111 such that the tooth is asymmetrical with respect to the first and second planes described above.

As shown in Figures 8a - 8f, the sides 116, 117, 119 may be planar. Alternatively, as shown in Figures 8g - 8j, the sides 116, 119 may be convex or concave.

Any one or more combinations of the tooth shapes identified in Figure 8a to Figure 8j may be incorporated into the toothed profile of the inner cushion of the present invention. Alternatively, the toothed profile 111 may include teeth with other similar shapes to those described above and with reference to the accompanying drawings.

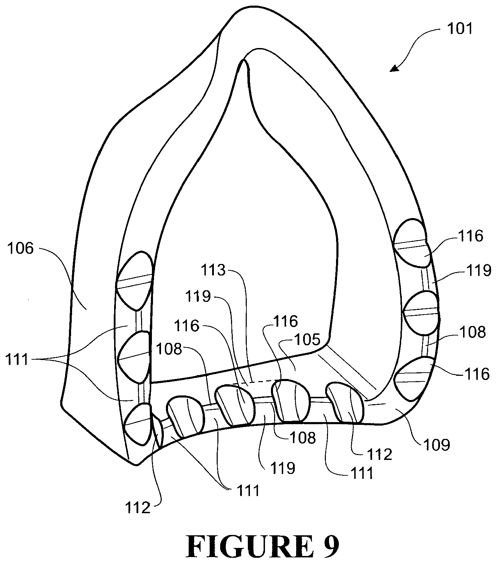

A preferred embodiment of the inner cushion of the present invention is shown in Figure 9. The preferred toothed profile 111 of Figure 9 includes teeth 111 similar in shape to the tooth of Figure 8h. However, the tooth profiles of Figures 8a to 8i are generally aligned centrally on the edge 109 of the inner cushion 101, as in the embodiments of Figures 5a and 5b. In the preferred embodiment of Figure 9, the toothed profile 110 in the chin region 56 (refer to Figure 4a) of the cushion 101 is formed by valleys 112 formed through the face side area 109 and inside surface 105 of the perimeter wall of the inner cushion. The valley portions 112 do not extend significantly through the outside surface 106 of the perimeter wall of the inner cushion. The teeth 111 in the chin region are therefore aligned inwards towards a centre of the inner cushion; one side 119 of the tooth 111 being coterminous with the inner surface 105 of the inner cushion, the other side 119 of the tooth being coterminous with the surface of edge 109. The apex area 108 of the tooth 111 is formed where the inner surface 105 meets the surface of edge 109. Sides 116 of the tooth 111 are coterminous with sides of the valley portions 112 on either side of the tooth 111.

The teeth 111 formed in the lower section of the cheek regions 55 of the cushion in Figure 9 are formed in a similar way to the teeth in the chin region. Valley portions 112 are formed in the edge 109 without valley portions 112 significantly extending through the outer surface 106 of the cushion 101. Due to a curved shape of edge 109 in the lower cheek region, the apex 108 is located more outwardly compared to the apex 108 of the teeth 111 in the chin region 56, and sides 119 of the teeth in the lower cheek regions are coterminous with the curved surface of edge 109 of the cushion, as shown in Figure 9.

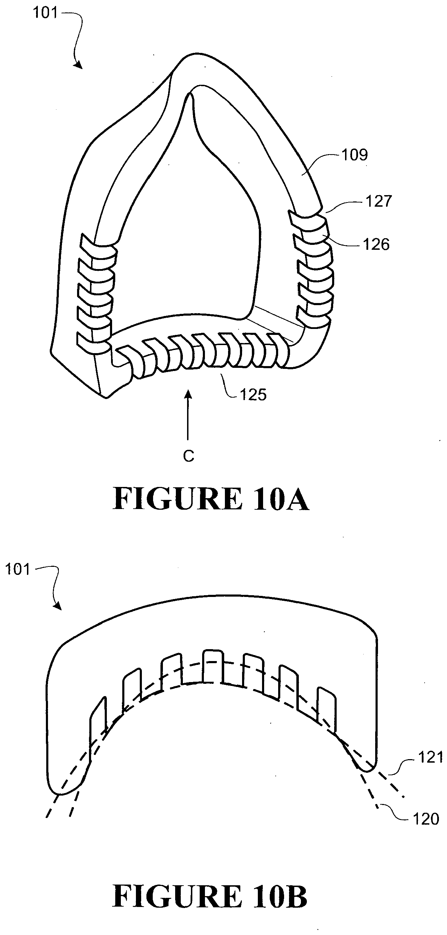

In the alternative embodiment of Figure 10, the toothed profile consists of a comb type profile, where the bearing surface area of the edge 109 of the cushion is reduced by forming a series of teeth, or fingers, 125 in the edge 109. In one embodiment, the width of each tooth 126 is approximately equal to the width of each gap 127 between each tooth 126. In this embodiment, the bearing surface area of the cushion edge 109 is halved to reduce the compression force required to conform the cushion to the facial profile of a user. Alternatively, each gap 127 may be wider than each tooth 126 to decrease the bearing surface area even further. In an alternative embodiment, the width of each tooth may be wider than the width of each gap 127. In a further alternative embodiment, the tooth width and the gap width may vary for different sections of the edge 109 of the cushion, to result in portions of the cushion that are more easily compressed compared to others portions.

For example, gaps 127 in the nasal bridge region may be more frequent than in other regions of the sealing interface, to reduce the sealing force on the bridge of the user's nose. Gaps 127 may be larger in the nasal bridge region than in other regions of the sealing interface, to reduce the sealing force on the bridge of the user's nose.

The width of gaps 127 or the widths of teeth 126 may vary around the perimeter of the sealing interface.

The width of the tooth or finger 125 may be chosen to be thin enough to allow buckling of the tooth under normal compression forces achieved during use. As the teeth 125 buckle under normal loads, the teeth provide a reduced level of support to the outer sheath.

Preferably the sealing interface of the present invention has an inner cushion with any one of the toothed profiles described above in the chin region of the cushion. Preferably the toothed profile consists of four teeth 111 or five valley portions 112 in the chin region, as shown in Figure 9. Preferably the sealing interface of the present invention has an inner cushion with any one of the toothed profiles described above extending into a lower section of each cheek portion of the cushion. Preferably the toothed profile consists of two teeth in the lower portion of each cheek region, as shown in Figure 9.

Alternatively, the toothed profile may extend around the full perimeter of the cushion. In a further alternative embodiment, a cushion may incorporate a toothed profile in the nasal bridge region.

Preferably the toothed profile has multiple teeth, with a distance between the apexes of adjacent teeth of approximately 5mm to 25mm. Preferably the distance between the apexes of adjacent teeth is less than 20mm. Most preferably the distance between adjacent tooth apexes is less than 15mm. The depth of a tooth 111, that is the distance between the apex 114 and the base 113, is approximately 3mm to 10mm. Preferably the tooth depth is 5mm to 10mm. The width of the base 113 of a tooth 111 is approximately 2mm to 25mm. Preferably the width of the base 113 of a tooth 111 is approximately 2mm to 20mm. Most preferably, the width of the base 113 of a tooth 111 is less than 15mm.

The angle of convergence 115 as shown in Figure 8k, must be high enough to ensure that a significant reduction in bearing surface area of the side 109 of the cushion that faces a user's face in use is achieved. For example, an angle of convergence 115 of zero means that no teeth are formed in the side 109 of the cushion 101. Preferably the angle of convergence is greater than 35 degrees. Most preferably the angle of convergence is greater than 45 degrees.

The distance between the apexes of adjacent teeth may be described as the tooth pitch of the toothed profile. In the preferred embodiment of the present invention, the ratio of the tooth depth over the tooth pitch is at least approximately 0.3. That is, the pitch is less than approximately three times the depth. Most preferably the ratio of the depth and pitch is at least 0.5, that is, the pitch is less than approximately twice the depth.

The cushion 101 preferably has a toothed profile 110 with a decreasing tooth depth as the toothed profile 110 extends from a central position of the toothed profile portion to an edge of the toothed profile portion, as shown in Figure 6a.

The dimensions of the teeth of the toothed profile described above are important to achieve an improved seal. In order to seal in and around finer features and detailed contours of a user's face, the relatively small pitch and high depth of the teeth perform well in sealing into creases and lines in a user's face, such as smile lines and creases around a user's mouth and between a user's nose and cheek area.

The present invention may be implemented in a mask assembly having an integral cushion and outer sheath. However, the embodiments having an integrally formed cushion and outer sheath may not preferred, as the amount of movement between the outer sheath and the inner cushion is limited compared to when a separate cushion and outer sheath is used. Having a separate sheath and cushion provides movement between the sheath and the cushion resulting in an improved fit compared to if the sheath and cushion are secured together.

### Plurality of spaced apart inner cushions

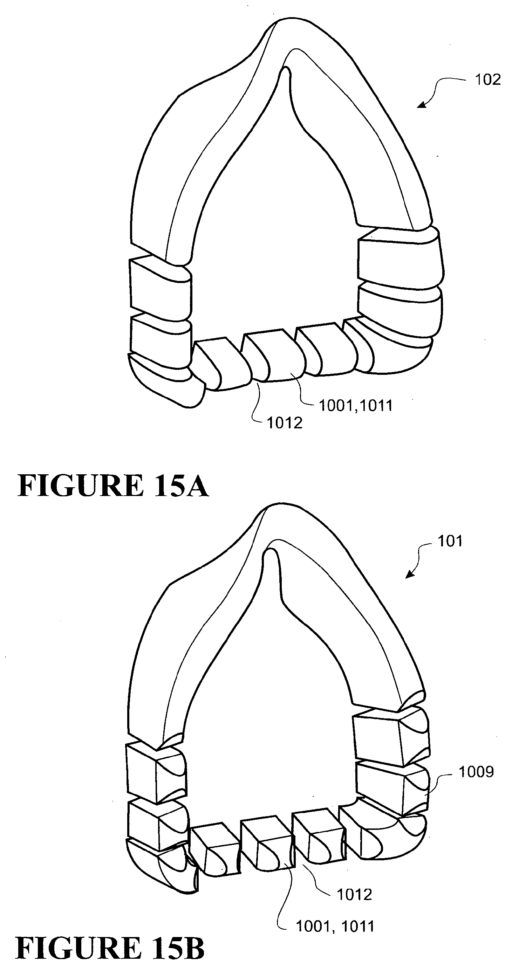

In an alternative embodiment of the present invention, shown in Figures 15a and 15b, the mask cushion 101 may be a plurality of cushions 1001 spaced apart to achieve a toothed profile comprising teeth 1011 and gaps 1012 between teeth 1011.

Each cushion 1011 has a side 1009 that bears against the user's face in use. As shown in Figure 15b, side 1009 of at least one cushion 1011 may be shaped as previously described in relation to Figures 8a to 8j, or any other toothed profile described herein.

The cushion may comprise a plurality of cushions in the chin or upper lip region, the cheek regions, nasal bridge region, or completely around the perimeter of the mask seal 100.

The embodiment of Figure 15a is similar in concept and operation to the embodiment shown in Figures 10a and 10b. For example, the removed portions 127 of the cushion of Figure 10a may be removed to a sufficient depth such that under normal operation, the mask seal 100 is not compressed further than the depth of the removed portions 127.

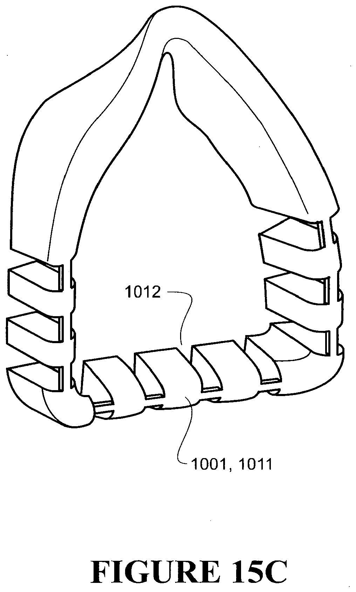

In the alternative embodiment of Figure 15c, the plurality of cushions may be attached to one another to form a single cushion comprising cushion elements 1001 joined together by joining elements 1013. Cushion elements 1001 are sized to extend between the mask hollow body 22 and an inner surface of the side of the outer sheath that bears against a user's face in use. Joining elements are sized to not extend between the hollow body 22 and the side of the outer sheath that bears against a user's face in use. Joining elements 1013 may be located to contact the outer sheath. For example, joining elements 1013 may contact an inner surface of the side of the outer sheath that seals against a user's face in use. In this case, joining elements are sufficiently thin to result in a low force for a given amount of seal compression at a joining element compared to the force required to compress the seal by the same amount of compression at a cushion element 1001. Alternatively, joining elements may be located to not contact the outer sheath.

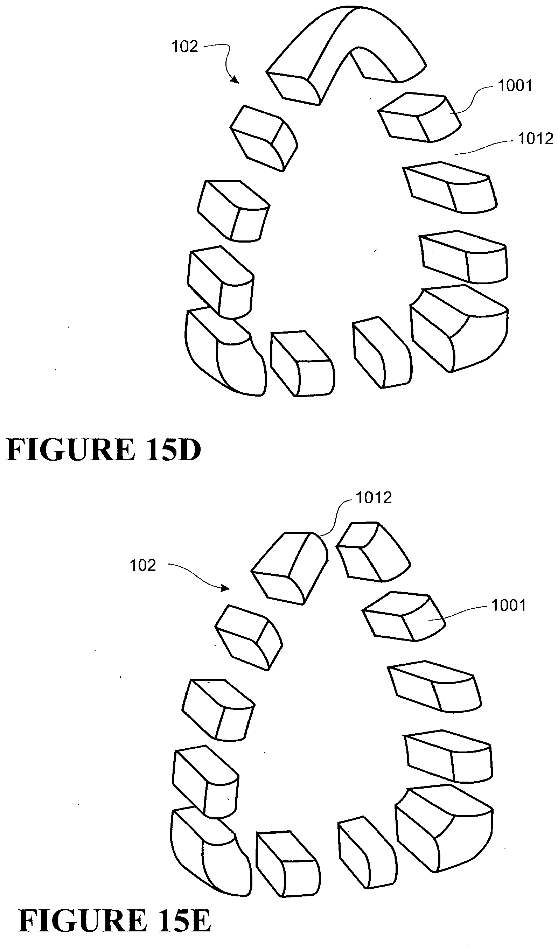

Further embodiments are illustrated in Figure 15d and 15e. In the embodiment of 15d, the inner cushion 102 comprises a plurality of cushions 1001 spaced apart around the full perimeter of the sealing interface, with gaps 1012 between adjacent cushions 1001. In the embodiment of Figure 15e, the individual cushions 1001 are arranged such that a gap 1012 is located in the nasal bridge region of the sealing interface.

The perimeter length of gaps 1012 between the plurality of cushions 1001 may be varied to result in portions of the cushion that are more easily compressed compared to others portions.

### Hole Profile

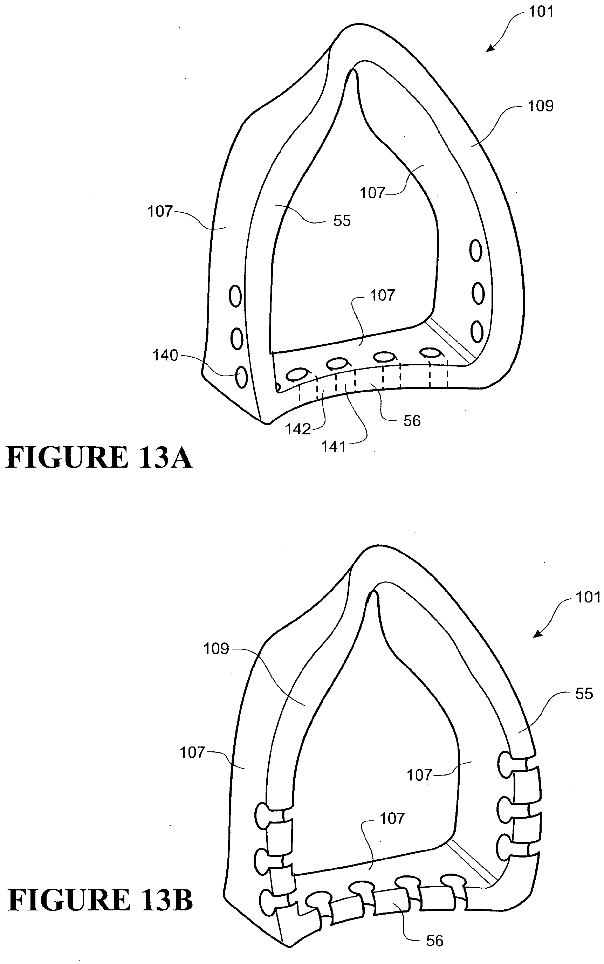

In a further alternative embodiment as shown in Figure 13b, a toothed profile in the face side of the inner cushion is achieved by holes 140 through the wall 107 of the inner cushion. The holes break the surface of the face side 109 of the inner cushion.

In the alternative embodiment of Figure 13a, holes 140 through the wall 107 of the inner cushion do not break the surface of the face side of the cushion. The holes 140 are provided near side 109. Portions 141 adjacent to the holes compress more easily compared to portions 142 located between adjacent holes. The holes may be circular or other shape such as oval as shown in Figure 13a.

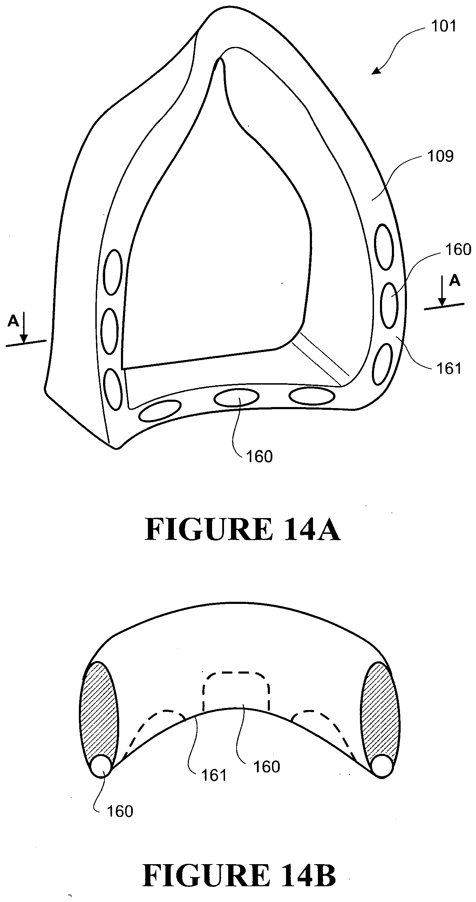

In an alternative embodiment of Figure 14a and 14b, holes or cavities 160 are provided in the side 109 of the cushion that bears against a user's face in use. Cavities result in portions of the seal 100 that compress more easily compared to portions of the seal at positions of the cushion 161 in between cavities 160.

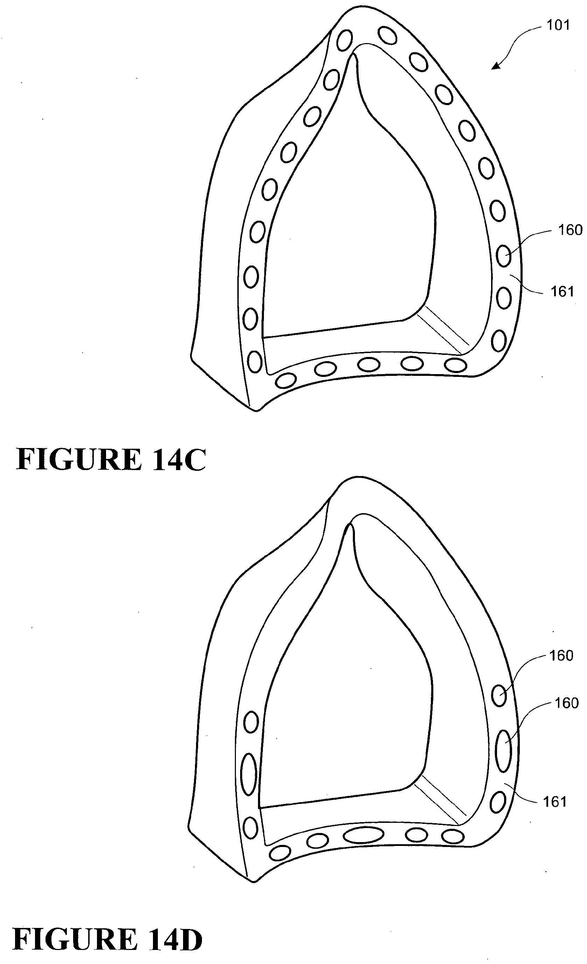

Cavities 160 may vary in perimeter length around the perimeter of the inner cushion to result in portions of the cushion perimeter that are more easily compressed compared to others portions, as shown in Figure 14d. Cavities 160 may vary in depth around the perimeter of the inner cushion to result in portions of the cushion that are more easily compressed compared to others portions, as shown in Figure 14b. Cavities 160 may vary in width across the side 109 of the cushion around the perimeter of the inner cushion to result in portions of the cushion that are more easily compressed compared to others portions, as indicated in Figure 14d.

The areas 161 between and around cavities 160 may form a continuous contact surface in contact with the outer sheath.

Cavities 160 may have an asymmetric shape as indicated in the cross sectional view of Figure 14b.

Cavities 160 may be positioned around the full perimeter of the cushion, each cavity being spaced apart by areas 161 between adjacent cavities, as shown in Figure 14c. Alternatively, cavities may only be provided in particular regions of the cushion. For example, cavities may be provided in the upper lip region of the cushion.

The perimeter length of spacing 161 between cavities may be varied. Alternatively, spacing between cavities may have equal length around the perimeter of the inner cushion.

A patient sealing interface comprising an inner cushion with a toothed profile on the face side of the inner cushion supporting an outer sheath achieves an improved sealing interface having a reduced leak rate for a given patient interface headgear strap tension compared to prior art patient sealing interfaces.

The invention has been described with reference to a number of embodiments. It is to be understood that these embodiments are merely illustrative. Modifications may be made thereto without departing from the scope of the invention.

## Claims

1. A sealing interface (100) for use as part of an apparatus for supplying a flow of respiratory gases to a user comprising:
a resilient rubber outer sheath (102) and a separate foam or gel inner cushion (101), in use the outer sheath (102) covering the inner cushion, the outer sheath (102) having a sheath first side, the sheath first side substantially sealing against a user's face, a face side (109) of the inner cushion (101) resiliently supporting at least a portion of the sheath first side, wherein:
the inner cushion (101) has a toothed profile (110) comprising a plurality of teeth (111) formed in the face side (109) of the inner cushion (101), each tooth (111) having an apex (114) between two valley portions (112) in the perimeter of the face side (109) of the inner cushion (101), in use the apex (114) being in supporting contact with the outer sheath first side.

2. The sealing interface of claim 1, wherein, in use, the apex (114) provides contact support against the outer sheath (102) at positions around the periphery of the sealing interface (100).

3. The sealing interface (100) as claimed in claim 1, wherein the toothed profile (110) extends substantially around the full perimeter of the inner cushion face side (109).

4. The sealing interface (100) as claimed in claim 1, wherein the sheath first side and the inner cushion face side (109) have a nasal bridge region, either one of an upper lip region or a chin region and a left cheek region and a right cheek region extending between the nasal bridge region and the upper lip or chin region, and a said toothed profile (110) is in the upper lip region or chin region.

5. The sealing interface (100) as claimed in claim 4, wherein a said toothed profile (110) is in a lower portion of each of the left and right cheek regions.

6. The sealing interface (100) as claimed in claim 4, wherein a said toothed profile (110) is in nasal bridge region.

7. The sealing interface (100) as claimed in claim 1, wherein each tooth (111) has at least two converging sides (116), each said converging side having an angle of convergence (115), the angle of convergence being at least 30 degrees.

8. The sealing interface (100) as claimed in claim 7, wherein each tooth (111) has a depth, the depth being approximately 3mm to 10mm and each tooth has a base (113), the base being approximately 2mm to 20mm wide.

9. The sealing interface (100) as claimed in claim 1, wherein the teeth (111) are aligned inwards towards a centre of the inner cushion, a side (119) of each tooth being coterminous with the face side (109) of the inner cushion and an opposite side (119) of each tooth being conterminous with an inside surface (105) of a perimeter wall of the inner cushion, the apex (108) of each tooth being formed where the inside surface (105) of the perimeter wall meets the face side (109) of the inner cushion.

10. The sealing interface (100) as claimed in claim 9, wherein a distance between the apex (108, 114, 118) of adjacent teeth (111) is less than approximately 20mm, and each said tooth (111) has a depth, and a ratio of the tooth depth over the tooth pitch is at least approximately 0.3, wherein the pitch is the distance between the apexes of adjacent teeth.

11. The sealing interface (100) as claimed in claim 10, wherein the tooth depth decreases as the toothed profile extends from a central position of the toothed profile to a side of the toothed profile extending along the perimeter of the sealing interface.

12. The sealing interface (100) as claimed in any one of claims 1 to 11, wherein the inner cushion comprises a plurality of spaced apart cushions (1001), each said cushion (1001) being a tooth (1011) of the toothed profile (110).

13. A mask assembly for use as part of an apparatus for supplying a flow of respiratory gases to a user comprising:
a mask body (22) having an inlet (23) through which said flow of respiratory gases are provided to the interior of said mask body, the inlet adapted to in use be connected to a gases conduit, and
the sealing interface (100) of any preceding claim, coupled to the mask body.

## Patentansprüche

1. Eine Verschlussschnittstelle (100) zur Verwendung als Teil einer Vorrichtung zur Lieferung einer Strömung von Atemgasen zu einem Benutzer, umfassend:
Eine nachgiebige äußere Hülle (102) aus Gummi und ein separates Schaumstoff- oder Gel-Innenpolster (101), wobei die äußere Hülle (102) im Einsatz das Innenpolster abdeckt, wobei die äußere Hülle (102) eine erste Seite der Hülle aufweist, die erste Seite der Hülle im Wesentlichen gegen das Gesicht eines Benutzers abdichtet, wobei eine Vorderseite (109) des Innenpolsters (101) mindestens einen Abschnitt der ersten Seite der Hülle nachgiebig stützt, wobei:
Das Innenpolster (101) ein Zahnprofil (110) aufweist, das eine Vielzahl von Zähnen (111) umfasst, die in der Vorderseite (109) des Innenpolsters (101) gebildet sind, wobei jeder Zahn (111) einen Scheitelpunkt (114) zwischen zwei Talabschnitten (112) im Umfang der Vorderseite (109) des Innenpolsters (101) aufweist, wobei der Scheitelpunkt (114) im Einsatz in stützendem Kontakt mit der ersten Seite der äußeren Hülle ist.

2. Die Verschlussschnittstelle nach Anspruch 1, wobei, der Scheitelpunkt (114), im Einsatz, Kontaktabstützung gegen die äußere Hülle (102) in Positionen um den Umfang der Verschlussschnittstelle (100) bereitstellt.

3. Die Verschlussschnittstelle (100) wie in Anspruch 1 beansprucht, wobei sich das Zahnprofil (110) im Wesentlichen um den ganzen Umfang der Vorderseite (109) des Innenpolsters erstreckt.

4. Die Verschlussschnittstelle (100) wie in Anspruch 1 beansprucht, wobei die erste Seite der Hülle und die Vorderseite (109) des Innenpolsters einen Nasenrückenbereich, entweder einen eines Oberlippenbereichs oder eines Kinnbereichs und eines linken Wangenbereichs und eines rechten Wangenbereichs aufweisen, die sich zwischen dem Nasenrückenbereich und dem Oberlippen- oder Kinnbereich erstrecken, und ein Zahnprofil (110) im Oberlippenbereich oder Kinnbereich ist.

5. Die Verschlussschnittstelle (100) wie in Anspruch 4 beansprucht, wobei sich ein Zahnprofil (110) in einem unteren Abschnitt jedes der linken und rechten Wangenbereiche befindet.

6. Die Verschlussschnittstelle (100) wie in Anspruch 4 beansprucht, wobei sich ein Zahnprofil (110) im Nasenrückenbereich befindet.

7. Die Verschlussschnittstelle (100) wie in Anspruch 1 beansprucht, wobei jeder Zahn (111) mindestens zwei konvergierende Seiten (116) aufweist, wobei jede konvergierende Seite einen Konvergenzwinkel (115) aufweist, wobei der Konvergenzwinkel mindestens 30 Grad hat.

8. Die Verschlussschnittstelle (100) wie in Anspruch 7 beansprucht, wobei jeder Zahn (111) eine Tiefe aufweist, wobei die Tiefe ca. 3 mm bis 10 mm ist und jeder Zahn eine Basis (113) aufweist, wobei die Basis ca. 2 mm bis 20 mm breit ist.

9. Die Verschlussschnittstelle (100) wie in Anspruch 1 beansprucht, wobei die Zähne (111) nach innen in Richtung einer Mitte des Innenpolsters ausgerichtet sind, wobei eine Seite (119) jedes Zahns gemeinsame Grenzen mit der Vorderseite (109) des Innenpolsters hat, und eine entgegengesetzte Seite (119) jedes Zahns gemeinsame Grenzen mit einer inneren Oberfläche (105) einer Umfangswand des Innenpolsters hat, wobei der Scheitelpunkt (108) jedes Zahns gebildet ist, wo die innere Oberfläche (105) der Umfangswand mit der Vorderseite (109) des Innenpolsters zusammen trifft.

10. Die Verschlussschnittstelle (100) wie in Anspruch 9 beansprucht, wobei ein Abstand zwischen dem Scheitelpunkt (108, 114, 118) angrenzender Zähne (111) geringer als ca. 20 mm ist, und jeder Zahn (111) eine Tiefe aufweist, und ein Verhältnis der Zahntiefe über die Zahnteilung mindestens ca. 0,3 beträgt, wobei die Teilung der Abstand zwischen den Scheitelpunkten angrenzender Zähen ist.

11. Die Verschlussschnittstelle (100) wie in Anspruch 10 beansprucht, wobei die Zahntiefe abnimmt sowie sich das gezahnte Profil von einer mittigen Position des gezahnten Profils zu einer Seite des gezahnten Profils erstreckt, das sich entlang des Umfangs der Verschlussschnittstelle erstreckt.

12. Die Verschlussschnittstelle (100) wie in einem der Ansprüche 1 bis 11 beansprucht, wobei das Innenpolster eine Vielzahl beabstandeter Polster (1001) umfasst, wobei jedes Polster (1001) ein Zahn (1011) des Zahnprofils (110) ist.

13. Eine Maskenbaugruppe zur Verwendung als Teil einer Vorrichtung zur Lieferung einer Strömung von Atemgasen zu einem Benutzer, umfassend:
Einen Maskenkörper (22) mit einem Einlass (23) durch den die Strömung von Atemgasen dem Innenraum des Maskenkörpers bereitgestellt wird, wobei der Einlass, im Einsatz, angepasst ist, an eine Leitung von Gasen angeschlossen zu werden, und
die Verschlussschnittstelle (100) nach einem vorhergehenden Anspruch, die mit dem Maskenkörper gekoppelt ist.

## Revendications

1. Interface de fixation (100) destinée à être utilisée dans le cadre d'un appareil fournissant un flux de gaz respiratoires à un utilisateur, comprenant :
une gaine externe en caoutchouc souple (102) et un coussin interne en mousse ou gel séparé (101), de sorte que lors de l'utilisation, la gaine externe (102) couvre le coussin interne, la gaine externe (102) ayant un premier côté de gaine, le premier côté de gaine se fixant sensiblement contre le visage de l'utilisateur, un côté visage (109) du coussin interne (101) supportant élastiquement au moins une partie du premier côté de gaine,
le coussin interne (101) ayant un profil denté (110) comprenant une pluralité de dents (111) formées dans le côté visage (109) du coussin interne (101), chaque dent (111) ayant un sommet (114) entre deux parties en creux (112) sur le périmètre du côté visage (109) du coussin interne (101), de sorte que lors de l'utilisation, le sommet (114) soit en contact de support avec le premier côté de gaine externe.

2. Interface de fixation selon la revendication 1, dans laquelle, lors de l'utilisation, le sommet (114) offre un support de contact contre la gaine externe (102) dans des positions autour de la périphérie de l'interface de fixation (100).

3. Interface de fixation (100) selon la revendication 1, dans laquelle le profil denté (110) s'étend sensiblement autour du périmètre complet du côté visage de coussin interne (109).

4. Interface de fixation (100) selon la revendication 1, dans laquelle le premier côté de gaine et le côté visage de coussin interne (109) ont une région de pont nasal, une région de lèvre supérieure ou une région de menton, et une région de joue gauche et une région de joue droite s'étendant entre la région de pont nasal et la région de lèvre supérieure ou la région de menton, ledit profil denté (110) étant dans la région de lèvre supérieure ou la région de menton.

5. Interface de fixation (100) selon la revendication 4, dans laquelle ledit profil denté (110) est dans une partie inférieure de chacune des régions de joue gauche et de joue droite.

6. Interface de fixation (100) selon la revendication 4, dans laquelle ledit profil denté (110) est dans une région de pont nasal.

7. Interface de fixation (100) selon la revendication 1, dans laquelle chaque dent (111) a au moins deux côtés convergents (116), chaque côté convergent ayant un angle de convergence (115), l'angle de convergence étant au moins égal à 30 degrés.

8. Interface de fixation (100) selon la revendication 7, dans laquelle chaque dent (111) a une profondeur, la profondeur mesurant de 3 mm à 10 mm environ, et chaque dent ayant une base (113), la base mesurant de 2 mm à 20 mm de large environ.

9. Interface de fixation (100) selon la revendication 1, dans laquelle les dents (111) sont alignées vers l'intérieur vers un centre du coussin interne, un côté (119) de chaque dent étant coïncidant avec le côté visage (109) du coussin interne, et un côté opposé (119) de chaque dent étant coïncidant avec une surface intérieure (105) d'une paroi périphérique du coussin interne, le sommet (108) de chaque dent étant formé là où la surface intérieure (105) de la paroi périphérique rencontre le côté visage (109) du coussin interne.

10. Interface de fixation (100) selon la revendication 9, dans laquelle une distance entre le sommet (108, 114, 118) de dents adjacentes (111) est inférieure à 20 mm environ, chacune desdites dents (111) ayant une profondeur, et le rapport entre la profondeur de dent et le pas de dent étant au moins égal à 0,3 environ, le pas étant la distance entre les sommets de dents adjacentes.

11. Interface de fixation (100) selon la revendication 10, dans laquelle la profondeur de dent diminue au fur et à mesure que le profil denté s'étend d'une position centrale du profil dentée à un côté du profil denté s'étendant le long du périmètre de l'interface de fixation.

12. Interface de fixation (100) selon l'une quelconque des revendications 1 à 11, dans laquelle le coussin interne comprend une pluralité de coussins espacés (1001), chacun desdits coussins (1001) étant une dent (1011) du profil denté (110).

13. Ensemble masque destiné à être utilisé dans le cadre d'un appareil fournissant un flux de gaz respiratoires à un utilisateur, comprenant :
un corps de masque (22) comportant une entrée (23) par laquelle ledit flux de gaz respiratoires est fourni à l'intérieur dudit corps de masque, l'entrée étant conçue pour être connectée, lors de l'utilisation, à un conduit de gaz, et
l'interface de fixation (100) selon l'une quelconque des revendications précédentes étant couplée au corps de masque.
